# EUROPÄISCHE PATENTSCHRIFT

(11) **EP 3 025 757 B1**
(45) Veröffentlichungstag und Bekanntmachung des Hinweises auf die Patenterteilung: **02.08.2017**
(21) Anmeldenummer: 15190504.9
(22) Anmeldetag: 20.10.2015
(51) Int. Cl.: A61N 1/37, A61N 1/08, A61N 1/39

(54) **MRT-TAUGLICHES AKTIVES IMPLANTIERBARES GERÄT**
MRI CAPABLE ACTIVE IMPLANTABLE DEVICE
APPAREIL ACTIF IMPLANTABLE POUR MRT

(30) Priorität: 27.11.2014 US 201462085259 P
(43) Veröffentlichungstag der Anmeldung: 01.06.2016
(73) Patentinhaber: BIOTRONIK SE & Co. KG, 12359 Berlin (DE)
(72) Erfinder: Dörr, Thomas, 12437 Berlin (DE)
(74) Vertreter: Galander, Marcus

(56) Entgegenhaltungen:
- EP-A1- 2 338 559
- EP-A2- 2 338 560
- US-A1- 2013 317 345

## Beschreibung

Die Erfindung betrifft einen MRT-Sensor für ein implantierbares elektronisches Gerät, der in der Lage ist zu erfassen, wenn das implantierbare elektronische Gerät innerhalb oder in unmittelbarer Umgebung eines MRT-Gerätes (Magnetresonanztomographen) betrieben wird. Außerdem betrifft die Erfindung ein MRT-taugliches implantierbares elektronisches Gerät, insbesondere ein implantierbares Herz-Therapie- und/oder Herz-Monitoring-Gerät, beispielsweise einen Herzschrittmacher oder Kardioverter/Defibrillator, mit einem MRT-Sensor.

Implantierbare Herz-Therapie- und/oder Herz-Monitoring-Geräte, z. B. Herzstimulatoren in Form von Herzschrittmachern oder Cardiovertern/Defibrillatoren, sind grundsätzlich bekannt. Solche Herzstimulatoren sind in der Regel an Elektrodenleitungen angeschlossen, die in einer Kammer eines Herzens oder in deren unmittelbarer Nähe Stimulations- und optional zusätzliche Defibrillationselektroden besitzen. Über eine Stimulationselektrode - genauer: einen oder mehrere Stimulationselektrodenpole - kann ein Herzschrittmacher einen elektrischen Stimulationsimpuls an das Muskelgewebe einer Herzkammer abgeben, um so eine stimulierte Kontraktion der Herzkammer hervorzurufen, sofern der Stimulationsimpuls eine ausreichende Intensität besitzt und das Herzmuskelgewebe (Myokard) sich nicht gerade in einer refraktären Phase befindet. Eine derartig stimulierte Kontraktion einer Herzkammer wird im Rahmen dieser Beschreibung als stimuliertes Ereignis bezeichnet, ein Stimulationsimpuls, der eine ausreichende Intensität besitzt, eine stimulierte Kontraktion einer Herzkammer hervorzurufen, wird als "überschwellig" bezeichnet. Kommt es zu einer natürlichen Kontraktion der Herzkammer, wird dies im Rahmen dieser Beschreibung als Eigenaktion oder als natürliches oder intrinsisches Ereignis bezeichnet. Eine Kontraktion, beispielsweise des rechten Atriums eines Herzens, wird als atriales Ereignis bezeichnet, welches beispielsweise ein natürliches atriales Ereignis sein kann, oder - im Falle eines atrialen Herzschrittmachers - auch ein stimuliertes atriales Ereignis. Im gleichen Sinne können natürliche (intrinsische) und stimulierte linksventrikuläre und rechtsventrikuläre Ereignisse unterschieden werden.

Eine lokale Erregung des Myokards breitet sich vom Erregungsort ausgehend per Reizleitung im Myokard aus und führt zu einer Depolarisation der Muskelzellen und damit zu einer Kontraktion des Myokards. Nach kurzer Zeit kommt es zu einer Repolarisation der Muskelzellen und damit zu einer Entspannung des Myokards. Während der Phase der Depolarisation sind die Herzmuskelzellen für eine Erregung unempfindlich, d.h. refraktär. Diese Zeit wird als Refraktärzeit bezeichnet. Die mit der Depolarisation und Repolarisation einhergehenden elektrischen Potenziale können wahrgenommen und deren zeitlicher Verlauf - als Elektrokardiogramm bezeichnet - ausgewertet werden.

In einem Elektrokardiogramm sind mit einer Kontraktion des Ventrikels einhergehende, eine Depolarisation der Herzmuskelzellen widerspiegelnde Aktionspotenziale als sogenannte Q-Zacke zu erkennen, während sich die mit der Entspannung des Myokards einhergehende Repolarisierung der Herzmuskelzellen in einer sogenannten T-Welle widerspiegelt.

Beim gesunden Menschen wird der jeweilige Herzrhythmus durch den vom autonomen Nervensystem gesteuerten Sinusknoten bestimmt. Dieser erregt per Reizleitung das rechte Atrium eines menschlichen Herzens und weiter über den AV-Knoten den (rechten) Ventrikel des Herzens. Ein vom Sinusknoten ausgehender natürlicher Herzrhythmus wird daher auch als Sinusrhythmus bezeichnet und führt zu jeweils natürlichen Kontraktionen der jeweiligen Herzkammer, die als natürliche (intrinsische) Ereignisse erfasst werden können.

Das Erfassen solcher natürlicher (intrinsischer) Ereignisse erfolgt durch Ableiten der elektrischen Potenziale des Myokards der jeweiligen Herzkammer mit Hilfe von Wahrnehmungselektroden, die Teil einer entsprechenden Elektrodenleitung sind. Dabei können die Wahrnehmungselektrodenpole gleichzeitig die Stimulationselektrodenpole sein und abwechselnd als Stimulations- und als Wahrnehmungselektrodenpol verwendet werden. Typischerweise ist für das Sensing - d.h. die Wahrnehmung intrinsischer Ereignisse - ein Wahrnehmungselektrodenpolpaar vorgesehen, das von zwei benachbarten Elektrodenpolen, nämlich einer Spitzenelektrode (Tip-Elektrode) und einer Ring-Elektrode, gebildet ist, von denen die Spitzenelektrode auch als Stimulationselektrodenpol dient. Auf diese Weise erfolgt eine bipolare Ableitung eines intrakardialen Elektrokardiogramms (IEGM). Dabei erfolgt die Wahrnehmung intrinsischer Ereignisse und die Stimulation im Ventrikel mit Hilfe einer ventrikulären Elektrodenleitung und die Stimulation und die Wahrnehmung intrinsischer Ereignisse im Atrium (im rechten Atrium) mit einer atrialen Elektrodenleitung, die separat an den jeweiligen Herzstimulator angeschlossen sind. Zusätzlich kann auch eine linksventrikuläre Elektrodenleitung vorgesehen sein, die typischerweise über den Coronarsinus und eine von diesem abzweigende Lateralvene in die Nähe des linken Ventrikels ragt und dort eine kleinflächige Stimulations- und/oder Wahrnehmungselektrode aufweisen kann.

Um den unterschiedlichen Bedürfnissen verschiedener Patienten gerecht werden zu können, können implantierbare Herzstimulatoren in verschiedenen Betriebsmodi betrieben werden. Die verschiedenen Stimulations- und Wahrnehmungsmodi werden in der Regel einheitlich mit einem Drei-Buchstaben-Code bezeichnet, von denen der erste Buchstabe den Stimulationsort bezeichnet (V = Ventrikel, A = Atrium, D = Ventrikel und Atrium), der zweite Buchstabe den Ort der Wahrnehmung bezeichnet (V = Ventrikel, A = Atrium, D = Ventrikel und Atrium) und der dritte Buchstabe die Betriebsart (I = Inhibiert, T = Getriggert, D = sowohl Inhibiert als auch Getriggert). Insbesondere für Zwei-Kammer-Herzschrittmacher im DDD-Modus ist es auch bekannt, eine ventrikuläre Stimulation synchron zu einer möglichst natürlichen atrialen Herzrate vorzunehmen. Falls im Atrium keine gesunde, natürliche Herzrate wahrzunehmen ist, beispielsweise im Falle einer atrialen Tachykardie oder einer atrialen Fibrillation, weisen grundsätzlich atriumsynchrone Herzschrittmacher häufig ein Modeswitching von einer atriumsynchronen ventrikulären Stimulation zu einer atriumasynchronen Stimulation im VVI-Modus auf, falls eine wahrgenommene atriale Rate außerhalb zulässiger Grenzen liegt. Weiterhin ist es bekannt, ventrikuläre Tachykardien im Rahmen einer Kardioversionstherapie durch Stimulation mit einer Stimulationsrate, die über der Tachykardierate liegt, zu behandeln.

Die Stimulationsmodi können durch entsprechende Steuerprogramme, die z. B. erfasste Ereignisse verarbeiten oder ignorieren, oder durch Steuerparameter eingestellt werden. So kann beispielsweise durch einen Steuerparameter die Erfassung von Ereignissen im Atrium und/oder Ventrikel aktiviert oder deaktiviert werden.

In Bezug auf die hierin verwendeten Bezeichnungen sei darauf hingewiesen, dass im Rahmen dieses Textes mit den Begriffen Stimulations- oder Wahrnehmungselektrode ein jeweiliger Elektrodenpol an einer Elektrodenleitung gemeint ist, also derjenige Teil einer Elektrodenleitung, über den Stimulationsimpulse abgegeben oder elektrische Potenziale aufgenommen werden. Es sei auch darauf hingewiesen, dass es auch üblich ist, mit "Stimulationselektrode" eine der Stimulation dienende Elektrodenleitung zu bezeichnen.

Die Wahrnehmungselektrodenpole sind im Betrieb des Herzstimulators mit entsprechenden Wahrnehmungseinheiten verbunden, die ausgebildet sind, ein jeweiliges über einen Wahrnehmungselektrodenpol (bzw. ein Wahrnehmungselektrodenpolpaar) aufgenommenes Elektrokardiogramm auszuwerten und insbesondere intrinsische atriale bzw. ventrikuläre Ereignisse zu detektieren, d.h. natürliche atriale oder ventrikuläre Kontraktionen. Dies geschieht beispielsweise durch einen Schwellwertvergleich, d.h. ein intrinsisches Ereignis wird detektiert, wenn ein jeweiliges intrakardiales Elektrokardiogramm einen als geeignet vorgegebenen Schwellwert überschreitet.

Aus der Frequenz, mit der atriale bzw. ventrikuläre Ereignisse aufeinander folgen, kann die jeweilige intrinsische atriale Herzrate (atriale Frequenz) bzw. ventrikuläre Herzrate (Ventrikelfrequenz) abgeleitet und somit beispielsweise Tachykardien detektiert werden.

Das Erfassen natürlicher Ereignisse dient bei bekannten Demand-Schrittmachern außerdem der Unterdrückung (Inhibierung) der Abgabe von Stimulationsimpulsen an eine entsprechende Herzkammer, falls das natürliche Ereignis in einem Zeitfenster vor der geplanten Abgabe eines Stimulationsimpulses an diese Herzkammer erfasst wird. Bei ratenadaptiven Herzschrittmachern wird der Zeitpunkt der Abgabe eines jeweiligen Stimulationsimpulses in Abhängigkeit einer jeweiligen Stimulationsrate geplant, die dem physiologischen Bedarf eines Patienten entsprechen soll, also bei größerer Anstrengung beispielsweise höher ist. Hierzu kann ein Herzstimulator mit einem oder mehreren Aktivitätssensoren ausgestattet sein, der beispielsweise ein CLS-Sensor (CLS: Closed Loop Stimulation) sein kann.

Problematisch ist, dass solche implantierbaren elektrischen medizinischen Geräte wie Herzstimulatoren durch starke elektromagnetische Felder oder Magnetfelder, wie sie zum Beispiel in einem Magnetresonanztomographen, (MRT-Gerät) auftreten, in ihrer Funktion stark beeinträchtigt werden können. Daher sind viele Träger von aktiven implantierbaren medizinischen Geräten (im Folgenden auch Implantat oder IMD genannt) für MRT-Untersuchungen kontraindiziert, obwohl MRT-Untersuchungen einen immer höheren Stellenwert in der diagnostischen Medizin erhalten.

Um Trägern von aktiven implantierbaren medizinischen Geräten MRT-Untersuchungen dennoch zu ermöglichen sind verschiedene Ansätze, die sich entweder auf die Durchführung der MRT-Untersuchung oder auf das implantierbare medizinische Gerät beziehen, bekannt.

Unter anderem sind Technologien zur Erkennung von Magnetfeldern bekannt, die auf herkömmlichen Verfahren zur Magnetfelddetektion beruhen. So beschreibt die US 2008/0154342 ein Verfahren unter Ausnutzung eines GMR-Sensors (Giant Magnetic Resistance), um problematische Magnetfelder von MRT-Geräten zu erkennen.

Daneben sind auch andere MRT-Sensoren bekannt, insbesondere Magnetfeldsensoren, Gradientenfeldsensoren, Hochfrequenz-Feldsensoren, Lagesensoren, Geräusch- oder (Lorentz-)Vibrationssensoren oder Spannungsverlaufssensoren, die charakteristische Spannungsverläufe überwachen.

Auch ist es bekannt, dass beispielsweise ein implantierbarer Kardioverter/Defibrillator (ICD) vor einer MRT-Untersuchung durch einen Kardiologen in einen Betriebsmodus versetzt wird, der durch die im MRT herrschenden Magnetfelder nicht beeinträchtigt wird. Nach der MRT-Untersuchung durch einen Radiologen muss ein Kardiologe den ICD wieder in einen Betriebsmodus versetzen, der den Bedürfnissen des Patienten entspricht.

Die Patentanmeldung EP 2338560 A2 beschreibt einen implantierbaren Stimulator der in der Lage ist, durch MRT-Geräte erzeugte elektromagnetische Störungen zu erkennen. Dabei werden die von einem MRT ausgesendeten RF-Drehfelder mit einer RF-Antenne und einer RF-Kommunikationseinheit vom IMD detektiert und einer MRT-Erkennungseinheit mit einer Demodulationseinheit zugeführt, die dann die vom MRT ausgesendeten RF-Felder erkennt und daraufhin ein MRT-Erkennungssignal übermittelt. In der Patentanmeldung US 20130317345 A1 wird ein Magnetfeldsensor eingesetzt um einen implantierbaren Defibrillator in einen MRI-Modus zu versetzen, der es erlaubt, dass in einem MRT-Gerät ein Shock abgegeben werden kann.

Der Erfindung liegt die Aufgabe zugrunde eine Ergänzung oder eine Alternative zu bekannten MRT-Sensoren zu schaffen. Ein weiterer Aspekt ist ein alternatives oder ergänztes implantierbares elektronisches Gerät mit einem MRT-Sensor.

Alle im Folgenden genannten Beispiele und Ausführungen, die nicht unter den unabhängigen Anspruch 1 fallen, sind nicht Teil der Erfindung.

Erfindungsgemäß wird diese Aufgabe durch ein implantierbares elektronisches Gerät gelöst, das mindestens eine elektromagnetische Felderkennungseinheit aufweist, die mit wenigstens einer Antenne verbunden ist. Außerdem ist die Felderkennungseinheit ausgebildet, auf elektromagnetische Felder anzusprechen, die typischerweise in der Umgebung eines Patienten außerhalb der Umgebung eines MRT-Gerätes auftreten und in der Umgebung eines MRT-Gerätes abgeschirmt sind. Darüberhinaus ist die Felderkennungseinheit ausgebildet, die Abwesenheit solcher elektromagnetischer Felder anzuzeigen, die typischerweise in der Umgebung eines Patienten außerhalb der Umgebung eines MRT-Gerätes auftreten und in der Umgebung eines MRT-Gerätes abgeschirmt sind. Die Felderkennungseinheit kann wenigstens einen auf solche elektromagnetische Felder, wie sie typischerweise in der Umgebung eines Patienten außerhalb der Umgebung eines MRT-Gerätes auftreten und in der Umgebung eines MRT-Gerätes abgeschirmt sind, abgestimmten Empfänger enthalten. Vorzugsweise enthält die Felderkennungseinheit mehrere solche Empfänger. Wenn keiner dieser Empfänger in seinem jeweiligen Empfangsbereich eine Feldstärke oberhalb einer vorgegebenen Grenzfeldstärke anzeigt, signalisiert eine mit dem Empfänger oder den Empfängern verbundene Steuereinheit die Abwesenheit solcher elektromagnetischer Felder, die typischerweise in der Umgebung eines Patienten außerhalb der Umgebung eines MRT-Gerätes auftreten und in der Umgebung eines MRT-Gerätes abgeschirmt sind.

Alternativ kann die Felderkennungseinheit auch einen durchstimmbaren Empfänger als Breitbandfrequenzerkennungseinheit zum Scannen eines breiteren Frequenzbereichs aufweisen und mit einem Feld-Charakteristika-Speicher verbunden sein oder einen solchen aufweisen. Der Feld-Charakteristika-Speicher enthält charakteristische Merkmale von elektromagnetischen Feldern, die nur außerhalb der Umgebung eines MRT-Gerätes auftreten und in der Umgebung eines MRT-Gerätes abgeschirmt sind. Solche charakteristischen Merkmale können beispielsweise Frequenzen oder Frequenzbereiche elektromagnetischer Felder sein.

Die Felderkennungseinheit ist so ausgelegt, dass diese die Abwesenheit eines oder mehrerer normalerweise in der Patientenumgebung vorhandenen elektromagnetischen Felder erkennt und immer dann die MRI-Umgebung anzeigt, wenn alle Felder "verschwunden" sind.

Ein erfindungsgemäßer MRT-Sensor erlaubt es durch automatisches Erkennen der Umgebung eines MRT-Gerätes, die Tauglichkeit eines aktiven implantierbaren medizinischen Gerätes auch unter dem Einfluss von Magnetfeldern, wie sie für einen Magnetresonanztomographen (MRT) typisch sind, zu verbessern.

Der MRT-Sensor ist Teil eines elektronischen implantierbaren Gerätes in Form eines Herzstimulators, der eine Stimulationssteuereinheit und eine Speichereinheit aufweist. In der Speichereinheit ist Programminformation zu Steuerprogrammen und/oder Steuerparametern gespeichert, die die Funktion der Stimulationssteuereinheit steuern. Außerdem ist die Stimulationssteuereinheit ausgebildet, dann, wenn die Felderkennungseinheit die Abwesenheit solcher elektromagnetischer Felder anzeigt, die typischerweise in der Umgebung eines Patienten außerhalb der Umgebung eines MRT-Gerätes auftreten und in der Umgebung eines MRT-Gerätes abgeschirmt sind, solch ein Steuerprogramm oder solche Steuerparameter auszuwählen, die einen sicheren Betrieb des implantierbaren elektronischen Gerätes in der Umgebung eines MRT-Gerätes erlauben. Die auszuwählenden Steuerprogramme oder Steuerparameter sind beispielsweise solche, die den Wahrnehmungs- und/oder Stimulationsmodus auf V00 oder A00 festlegen, also die Wahrnehmung ausschalten.

Weiterhin ist die Stimulationssteuereinheit vorzugsweise ausgebildet das ausgewählte Steuerprogramm oder die ausgewählten Steuerparameter solange beizubehalten, solange die Felderkennungseinheit die Abwesenheit solcher elektromagnetischer Felder anzeigt, die typischerweise in der Umgebung eines Patienten außerhalb der Umgebung eines MRT-Gerätes auftreten und in der Umgebung eines MRT-Gerätes abgeschirmt sind.

Steuerprogramme oder Steuerparameter bestimmen die Funktionsweise, insbesondere den jeweiligen Betriebsmodus des implantierbaren elektronischen Gerätes, der typischerweise von der Stimulationssteuereinheit gesteuert wird. In diesem Sinne beeinflussen in der Speichereinheit gespeicherte Steuerprogramme oder Steuerparameter die Arbeitsweise der Stimulationssteuereinheit. Im vorliegenden Fall steuert die Stimulationssteuereinheit nicht nur den Betrieb des implantierbaren elektronischen Gerätes hinsichtlich beispielsweise einer Therapieabgabe wie der Herzstimulation, sondern die Stimulationssteuereinheit wählt auch ein jeweils anzuwendendes Steuerprogramm oder anzuwendende Steuerparameter aus, die ihrerseits dann wieder z. B. die Therapieabgabe definieren.

Die Erfindung ermöglicht den sicheren Betrieb eines elektronischen Implantates in einem MRT, ohne dass unmittelbar vor der Untersuchung eine Umprogrammierung bzw. Anpassung des MRT-Programmes stattfinden muss.

In einer bevorzugten Ausführungsform ist das elektronische implantierbare Gerät ein implantierbarer Pulsgenerator (IPG) und/oder ein implantierbarer Cardioverter/Defibrillator (ICD) und/oder ein Herzstimulator für eine kardiale Resynchronisationstherapie (CRT) oder ein Neurostimulator. Besonders bevorzugt ist das elektronische implantierbare Gerät ein Herz-Therapie- und/oder Herz-Monitoring-Gerät, insbesondere ein implantierbarer, beispielsweise biventrikulärer Herzschrittmacher und/oder Kardioverter/Defibrillator. Mit einem biventrikulären Herzschrittmacher kann insbesondere eine kardiale Resynchronisations-Therapie (CRT) durchgeführt werden.

Vorzugsweise enthält die Speichereinheit ein von der Steuereinheit wählbares Steuerprogramm, das so konfiguriert ist, dass dieses Steuerprogramm ein Auslösen von Stimulationsimpulsen unterbindet. Die Speichereinheit beinhaltet somit mindestens ein Programm, das einen Betriebsmodus bewirkt, der ohne Stimulation ist.

Falls das implantierbare elektronische Gerät ein Herzschrittmacher ist, kann die Speichereinheit alternativ auch ein von der Steuereinheit wählbares Steuerprogramm oder wählbare Steuerparameter enthalten, das so konfiguriert ist bzw. die so konfiguriert sind, dass es die Steuereinheit veranlasst, eine asynchrone Stimulation in einem asynchronen Stimulationsmodus wie V00 oder D00 zu bewirken. Gemäß dem weiter vorne beschriebenen Drei-Buchstaben-Code kommen diese Stimulationsmodi ohne Wahrnehmung intrinsischer Ereignisse aus, sodass auch keine Inhibierung oder Triggerung von Stimulationsimpulsen durch erfasste intrinsische Ereignisse erfolgt. Derartige Stimulationsmodi tragen dem Umstand Rechnung, dass ein Herzstimulator infolge wechselnder magnetischer oder elektromagnetischer Felder durch diese Felder induzierte Signale fälschlicherweise als intrinsische Ereignisse erfassen könnte. In einem reinen Stimulationsmodus, der ohne das Erfassen intrinsischer Ereignisse auskommt, besteht dieses Problem nicht.

Falls das implantierbare elektronische Gerät ein Herzschrittmacher ist, ist es weiter bevorzugt, wenn in der Speichereinheit ein Steuerprogramm abgelegt ist, das von der Steuereinheit gewählt werden kann und das so konfiguriert ist, dass es eine asynchrone Stimulation in einem asynchronen Stimulationsmodus wie VOO oder DOO bewirkt.

Jeweils auszuwählende Steuerprogramme oder Steuerparameter sind vorzugsweise grundsätzlich so gewählt, dass sie einen grundsätzlich sicheren Betrieb des implantierbaren elektronischen Gerätes in einem Magnetresonanztomographen gewährleisten, beispielsweise das Deaktivieren der Schockabgabe in einem ICD.

Vorzugsweise weist der MRT-Sensor zusätzlich einen Magnetfeldsensor und/oder einen Gradientenfeldsensor und/oder einen Hochfrequenz-Feldsensor und/oder einen Lagesensor und/oder einen (Lorenz-)Vibrationssensor und/oder einen Spannungsverlaufssensor auf, der charakteristische Spannungsverläufe beispielsweise in den Elektrodenleitungen überwacht.

Die Erfindung soll nun anhand von Ausführungsbeispielen mit Bezug auf die Figuren näher erläutert werden. Die Figuren zeigen in
- Figur 1:: Ein System mit einem Herz-Therapie- und/oder Herz-Monitoring-Gerät als implantierbarem elektronischen Gerät in Form eines Dreikammer-ICD-Systems;
- Figur 2:: die Hauptbestandteile des Herz-Therapie- und/oder Herz-Monitoring-Gerätes aus Figur 1;
- Figur 3:: einen typischen Ablauf einer MRT-Untersuchung eines ICD-Patienten mit einem implantierbaren elektronischen Gerät ohne MRT-Sensor; und
- Figur 4:: ein mögliches Blockschaltbild eines erfindungsgemäßen MRT-Sensors.

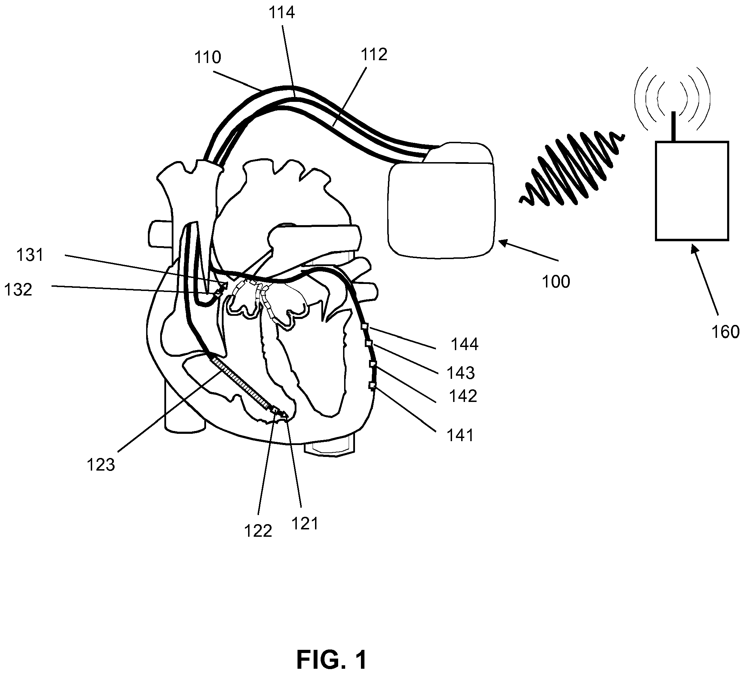

In Figur 1 ist als Beispiel für ein aktives implantierbares medizinisches Gerät ein Herz-Therapie- und/oder Herz-Monitoring-Gerät in Form eines Dreikammer-ICD-Systems dargestellt. Dieses umfasst einen Generator 100 (als das Herz-Therapie- und/oder Herz-Monitoring-Gerät), der mit mehreren implantierbaren Elektrodenleitungen 110, 112 und 114 verbunden ist. Zur rechtsventrikulären Wahrnehmung und Stimulation ist eine rechtsventrikuläre (RV-) Elektrodenleitung 110 vorgesehen, die eine rechtsventrikuläre (RV-) Tip-Elektrode 121 und eine rechtsventrikuläre (RV-) Ring-Elektrode 122 am distalen Ende umfasst. Über die RV-Tip-Elektrode 121 können im Betrieb bei Bedarf rechtsventrikuläre Stimulationsimpulse für eine biventrikuläre CRT-Stimulation abgegeben werden. Zur Defibrillationsschockabgabe sind an dieser Elektrodenleitung 110 eine distale Schockwendel 123 und optional auch eine proximale Schockwendel (nicht dargestellt) angebracht. Die Gegenelektrode bildet hier das Generatorgehäuse des Generators 100.

Eine rechtsatriale Elektrodenleitung 112 weist an ihrem distalen Ende einen bipolaren Wahrnehmungs- und Stimulationspol mit einer rechtsatrialen Tip-Elektrode 131 und einer rechtsatrialen Ring-Elektrode 132 auf und dient der Wahrnehmung des atrialen Rhythmus und bei Bedarf der atrialen Stimulation.

Außerdem umfasst das System auch eine linksventrikuläre CS-Elektrodenleitung zur Abgabe von linksventrikulären Stimulationsimpulsen zur CRT über einen oder mehrere von insgesamt vier linksventrikulären (CS-) Stimulationselektrodenpolen 141, 142, 143 und 144.

Für eine Kommunikation mit externen Programmier- und Steuer- und Datenübertragungsgeräten 160 ist in dem Generator 100 eine drahtlose, bidirektionale Telemetrieeinheit vorgesehen.

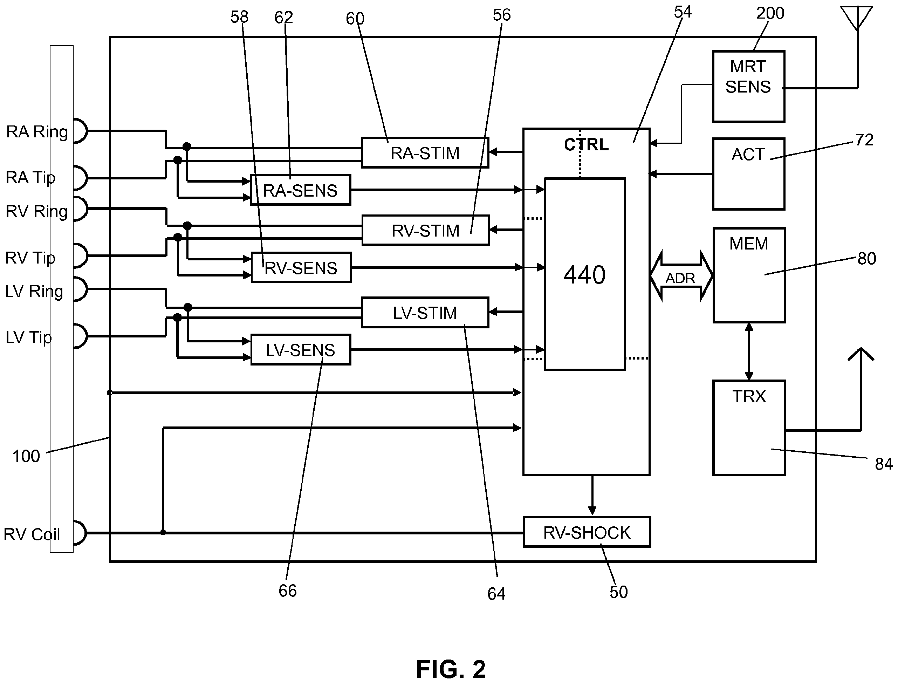

Figur 2 zeigt die Hauptbestandteile des Herzstimulators 100 aus Figur 1. Auf der linken Seite sind die elektrischen Anschlüsse für die verschiedenen Elektroden 121, 122, 131 und 132 dargestellt. Die Schockelektroden 123 sind mit einem rechtsventrikulären Schockimpulsgenerator 50 verbunden. Der Schockimpulsgenerator 50 ist mit einer Stimulationssteuereinheit 54 verbunden, die den Schockimpulsgenerator 50 bei Bedarf zur Erzeugung und Abgabe eines Defibrillationsschocks ansteuert.

Der Anschluss für die rechtsventrikuläre Tip-Elektrode 121 (RV Tip) sowie der Anschluss für die rechtsventrikuläre Ring-Elektrode 122 (RV Ring) ist jeweils sowohl mit einer rechtsventrikulären Stimulationseinheit 56 als auch mit einer rechtsventrikulären Wahrnehmungseinheit 58 verbunden. Sowohl die rechtsventrikuläre Stimulationseinheit 56 als auch die rechtsventrikuläre Wahrnehmungseinheit 58 sind jeweils mit der Stimulationssteuereinheit 54 verbunden.

Die rechtsventrikuläre Stimulationseinheit 56 ist dazu ausgebildet, auf ein Ansteuersignal der Stimulationssteuereinheit 54 hin einen rechtsventrikulären Stimulationsimpuls zu erzeugen und im Anschluss über die rechtsventrikuläre Ring-Elektrode 122 (RV Ring) und die rechtsventrikuläre Tip-Elektrode 121 (RV Tip) abzugeben. Alternativ ist es auch möglich, dass das Gehäuse des Generators 100 eine neutrale Elektrode bildet und die rechtsventrikuläre Stimulationseinheit 56 mit dem Anschluss für die rechtsventrikuläre Ring-Elektrode 122 (RV Ring) und dem Gehäuse als andere Elektrode zur Abgabe eines Stimulationsimpulses verbunden ist. Ein rechtsventrikulärer Stimulationsimpuls unterscheidet sich von einem Defibrillationsschock dadurch, dass der Stimulationsimpuls eine wesentlich geringere Impulsstärke besitzt, so dass er nicht wie ein Defibrillationsschock auf einen Schlag das vollständige Herzgewebe (Myokard) einer Herzkammer erregt, sondern nur die Herzmuskelzellen in unmittelbarer Umgebung der rechtsventrikulären Tip-Elektrode 121 (RV Tip). Diese Erregung breitet sich dann durch natürliche Reizleitung über den gesamten rechten Ventrikel weiter aus und sorgt so für eine stimulierte Kontraktion des rechten Ventrikels.

Die rechtsventrikuläre Wahrnehmungseinheit 58 ist dazu ausgebildet, an dem Anschluss für die rechtsventrikuläre Ring-Elektrode 122 (RV Ring) und die rechtsventrikuläre Tip-Elektrode 121 (RV Tip) anliegende elektrische Potenziale zunächst durch einen Eingangsverstärker zu verstärken und zu filtern. Weiterhin ist die rechtsventrikuläre Wahrnehmungseinheit 58 ausgebildet, den Verlauf der an ihren Eingängen anliegenden elektrischen Signale derart auszuwerten, dass die rechtsventrikuläre Wahrnehmungseinheit 58 selbsttätig eine natürliche (intrinsische), d.h. selbsttätige, Kontraktion des rechten Ventrikels detektiert. Dies kann beispielsweise dadurch geschehen, dass der Verlauf des an den Eingängen der rechtsventrikulären Wahrnehmungseinheit 58 anliegenden Signals mit einem Schwellenwert verglichen wird. Typischerweise ist die größte Amplitude des Signals in Form der sogenannten R-Zacke kennzeichnend für eine natürliche Kontraktion des rechten Ventrikels, die durch Schwellenwertvergleich detektiert werden kann. Die rechtsventrikuläre Wahrnehmungseinheit 58 gibt daraufhin ein entsprechendes, eine natürliche Kontraktion des rechten Ventrikels anzeigendes Ausgangssignal an die Stimulationssteuereinheit 54 aus. Der Zeitpunkt, an dem der Schwellenwert überschritten wird, ist der Erfassungszeitpunkt für das jeweilige Ereignis.

In gleicher Art und Weise können auch für die Schockelektroden eine oder mehrere (in Figur 2 nicht dargestellte) Wahrnehmungseinheiten vorgesehen sein. Diese Wahrnehmungseinheit oder Wahrnehmungseinheiten sind bevorzugt ausgebildet, Signale zwischen den Schockelektroden, zwischen der Schockelektrode 123 und dem Gehäuse des Generators 100 oder zwischen der anderen Schockelektrode und dem Gehäuse des Generators 100 zu erfassen.

In analoger Weise sind der Anschluss für die rechtsatriale Tip-Elektrode 131 (RA Tip) und der Anschluss für die rechtsatriale Ring-Elektrode 132 (RA Ring) sowohl mit einer rechtsatrialen Stimulationseinheit 60 als auch mit einer rechtsatrialen Wahrnehmungseinheit 62 verbunden, die jeweils ihrerseits wiederum mit der Stimulationssteuereinheit 54 verbunden sind. Die rechtsatriale Stimulationseinheit 60 ist dazu ausgebildet, Stimulationsimpulse zu erzeugen, deren Stärke ausreicht, das rechtsatriale Myokard zu erregen. Die rechtsatrialen Stimulationsimpulse können dabei eine andere Impulsstärke besitzen als die rechts-ventrikulären Stimulationsimpulse. Die rechtsatriale Wahrnehmungseinheit 62 ist ausgebildet, aus dem Verlauf des an ihren Eingängen anliegenden Differenzsignals eine sogenannte P-Welle zu detektieren, die eine natürliche (intrinsische) Kontraktion des rechten Atriums kennzeichnet. Detektiert die rechtsatriale Wahrnehmungseinheit 62 eine entsprechende P-Welle, erzeugt sie ein Ausgangssignal und gibt dieses an die Stimulationssteuereinheit 54 weiter, welches eine natürliche Kontraktion des rechten Atriums kennzeichnet.

In gleicher Weise sind auch der Anschluss für die linksventrikuläre Tip-Elektrode 141 (LV Tip) und die Anschlüsse für die linksventrikulären Ring-Elektroden 142, 143 und 144 (aus Vereinfachungsgründen ist nur ein Anschluss LV Ring dargestellt) mit einer linksventrikulären Stimulationseinheit 64 und einer linksventrikulären Wahrnehmungseinheit 66 verbunden. Die linksventrikuläre Stimulationseinheit 64 und die linksventrikuläre Wahrnehmungseinheit 66 sind ebenso mit der Stimulationssteuereinheit 54 verbunden. Beide funktionieren ähnlich wie die bereits beschriebenen Stimulationseinheiten 56 und 60 und Wahrnehmungseinheiten 58 und 62.

Als weiterer Bestandteil des Herzstimulators 100 ist ein Beschleunigungssensor 72 mit der Stimulationssteuereinheit 54 verbunden und in das Gehäuse des Herzstimulators 100 integriert. Der Beschleunigungssensor 72 ist dazu ausgebildet, ein von der körperlichen Aktivität eines Patienten abhängiges Bewegungssignal zu erfassen und ein entsprechendes, die körperliche Aktivität des Patienten anzeigendes erstes Akzelerometer-Ausgangssignal an die Stimulationssteuereinheit 54 auszugeben. Dies erlaubt es, dass die Stimulationssteuereinheit 54 das Timing der Stimulationsimpulse an den Bedarf des Patienten (hämodynamischen Bedarf) anpasst. Das Akzelerometer-Ausgangssignal kann auch zum Bestimmen von Ruhephasen genutzt werden, in denen bevorzugt eine Dislokationsdetektion stattfinden kann.

Weiterhin umfasst der Generator 100 eine Speichereinheit 80, die mit der Stimulationssteuereinheit 54 verbunden ist und es erlaubt, von der Stimulationssteuereinheit 54 erzeugte oder ausgewertete Signale zu speichern. Andererseits erlaubt es die Speichereinheit 80, Steuerprogramme für die Stimulationssteuereinheit 54 in veränderbarer Form zu speichern.

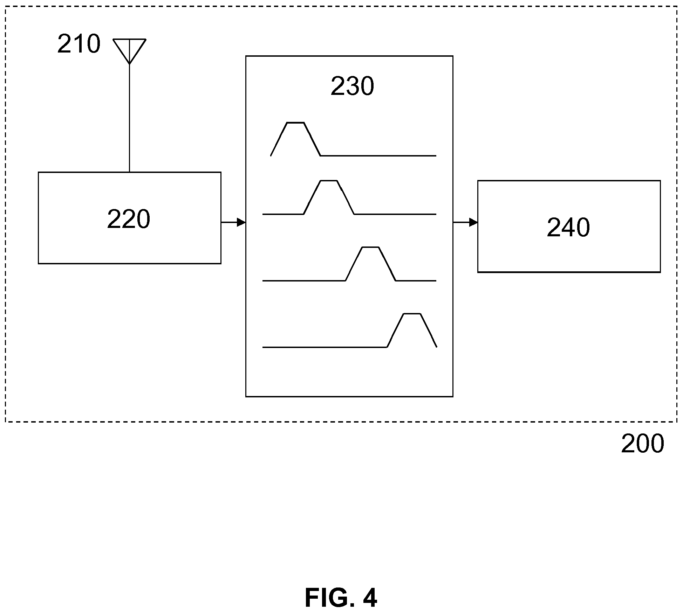

Des Weiteren ist die Stimulationssteuereinheit 54 mit einem MRT-Sensor 200 verbunden. Der MRT-Sensor 200 umfasst eine Felderkennungseinheit, wie sie im Zusammenhang mit Figur 4 näher erläutert ist und kann zusätzlich einen Magnetfeldsensor und/oder einen Gradientenfeldsensor und/oder einen Hochfrequenz-Feldsensor und/oder einen Lagesensor und/oder einen (Lorentz-)Vibrationssensor und/oder einen Spannungsverlaufssensor umfassen.

Die Speichereinheit 80 ist mit einer Telemetrieeinheit 84 verbunden, die es erlaubt, in der Speichereinheit 80 abgelegte Daten drahtlos an das externe Gerät 160 zu übertragen oder Programmierbefehle seitens des externen Geräts 160 zu dem Herzstimulator 100 zu übertragen und in der Speichereinheit 80 zu speichern.

Vorzugsweise erlaubt das implantierbare elektronische Gerät eine automatische MRT-Erkennung und Umschaltung in einen sicheren Zustand während einer MRT-Untersuchung, wobei in dem implantierbaren elektronischen Gerät mindestens zwei 13.191P-EP / 28.11.2016 patientenindividuelle MRT-Programmeinstellungen hinterlegt sind, die in Abhängigkeit eines vom Implantat vor der MRT-Untersuchung erfassten Patientenstatus automatisch ausgewählt werden.

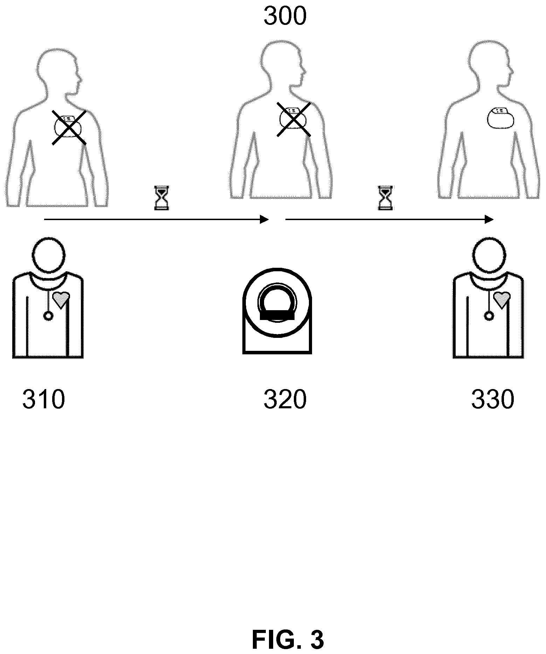

In Figur 3 ist ein typischer Ablauf einer MRT-Untersuchung eines ICD-Patienten ohne erfindungsgemäßes Gerät dargestellt. Ein ICD-Patient 300 wird vor der geplanten MRT-Untersuchung von einem Kardiologen 310 nachgesorgt und der ICD ausgeschaltet. Mit einer zeitlichen Verzögerung von Stunden bis wenigen Tagen erfolgt die MRT-Untersuchung bei einem Radiologen 320. Nach einer weiteren Verzögerung wird der Patient wieder vom Kardiologen 330 betreut und der ICD wieder eingeschaltet. Während der gesamten Zeit beginnend mit der MRT-Untersuchung durch einen Radiologen 320 bis hin zum Wiedereinschalten des ICD durch den Kardiologen 330 ist der Patient ohne den Schutz des implantierten Defibrillators und weitgehend ohne Rhythmusmonitoring. Derzeit wird dieses verbleibende Restrisiko, gemessen an dem Nutzen der MRT-Untersuchung in Kauf genommen.

Durch den Einsatz eines MRT-Sensors 200 kann der dargestellte Ablauf zwar derart verändert werden, dass nun die MRT-Einstellung nur unmittelbar während der MRT-Untersuchung wirksam wird und die abschließende Nachsorge durch den Kardiologen 330 zur Rückprogrammierung entfallen kann. Allerdings kann der Zeitpunkt der ersten Nachsorge durch den Kardiologen 310 zur Festlegung des MRT-Programmes nicht beliebig lange vor der eigentlichen MRT-Untersuchung durch den Radiologen 320 stattfinden, da sich die Konditionen des Patienten ändern und damit auch eine Anpassung des MRT-Programmes notwendig wird.

Als Beispiel sei hier die Notwendigkeit einer antibradykarden Stimulation genannt. Patienten, die keine Stimulation benötigen, sollten grundsätzlich im AUS-Mode im MRT untersucht werden, um so die Gefahr einer Arrhythmieinduktion zu vermeiden. Patienten, die eine Stimulation benötigen, müssen jedoch in einer asynchronen Betriebsart (VOO, DOO) im MRT stimuliert werden. Die jeweilige Schrittmacherabhängigkeit kann sich jedoch im Laufe der Zeit verändern.

In Figur 4 ist ein mögliches Blockschaltbild eines MRT-Sensors 200 eines solchen elektronischen Implantates 100 dargestellt. Der MRT-Sensor 200 beinhaltet mindestens eine Antenne 210, verbunden mit einem Antennenanpassungsnetzwerk/-verstärker 220, wiederum verbunden mit einer durchstimmbaren, stromarmen Breitbandfrequenzerkennungseinheit 230, die zyklisch die Feldstärken der definierten Umgebungsfrequenzen abscannt und an eine Steuereinheit 240 signalisiert. Die Steuereinheit 240 vergleicht alle von der Breitbandfrequenzerkennungseinheit 230 mit einer Feldstärke oberhalb einer (jeweils) vorgegebenen Grenzfeldstärke empfangenen Umgebungsfrequenzen mit zuvor gespeicherten Feld-Charakteristiken, nämlich insbesondere zuvor gespeicherten Frequenzbereichen solcher Umgebungsfrequenzen, die typischerweise in der Umgebung des implantierbaren elektronischen Gerätes außerhalb der Umgebung eines MRT-Gerätes vorhanden sind. Erkennt diese Steuereinheit 240 keine einzige dieser zuvor gespeicherten Umgebungsfrequenzen mehr, so signalisiert die Steuereinheit 240 eine MRT-Umgebung. Die Breitbandfrequenzerkennungseinheit 230 bildet zusammen mit der Steuereinheit 240 eine Felderkennungseinheit.

Geeignete Frequenzen sollten aus den Frequenzbändern zwischen 30kHz bis 30 GHz ausgewählt werden.

## Patentansprüche

1. Implantierbares elektronisches Gerät, das mindestens eine elektromagnetische Felderkennungseinheit (200) aufweist, die mit wenigstens einer Antenne verbunden und ausgebildet ist, auf elektromagnetische Felder anzusprechen, die typischerweise in der Umgebung eines Patienten außerhalb der Umgebung eines MRT-Gerätes auftreten und in der Umgebung eines MRT-Gerätes abgeschirmt sind, wobei die Felderkennungseinheit weiter ausgebildet ist, die Abwesenheit solcher elektromagnetischer Felder anzuzeigen, wobei das implantierbare elektronische Gerät ein Herzstimulator (100) ist, der eine Stimulationssteuereinheit (54) und eine Speichereinheit (80) aufweist, in der Programminformation zu Steuerprogrammen und/oder Steuerparametern gespeichert ist, die die Funktion der Stimulationssteuereinheit (54) steuern, **dadurch gekennzeichnet, dass** die Stimulationssteuereinheit (54) ausgebildet ist, dann, wenn die Felderkennungseinheit (200) die Abwesenheit solcher elektromagnetischer Felder anzeigt, die typischerweise in der Umgebung eines Patienten außerhalb der Umgebung eines MRT-Gerätes auftreten und in der Umgebung eines MRT-Gerätes abgeschirmt sind, solch ein Steuerprogramm oder solche Steuerparameter auszuwählen, die einen sicheren Betrieb des implantierbaren elektronischen Gerätes in der Umgebung eines MRT-Gerätes erlauben.

2. Implantierbares elektronisches Gerät gemäß Anspruch 1, **dadurch gekennzeichnet, dass** die Felderkennungseinheit (200) wenigstens einen auf solche elektromagnetische Felder, wie sie typischerweise in der Umgebung eines Patienten außerhalb der Umgebung eines MRT-Gerätes auftreten und in der Umgebung eines MRT-Gerätes abgeschirmt sind, abgestimmten Empfänger enthält.

3. Implantierbares elektronisches Gerät gemäß Anspruch 1, **dadurch gekennzeichnet, dass** die Felderkennungseinheit einen durchstimmbaren Empfänger (230) zum Scannen eines breiteren Frequenzbereichs aufweist und mit einem Feld-Charakteristika-Speicher verbunden ist oder einen solchen aufweist, wobei der Feld-Charakteristika-Speicher charakteristische Merkmale von 13.191P-EP / 28.11.2016 elektromagnetischen Feldern enthält, die nur außerhalb der Umgebung eines MRT-Gerätes auftreten und in der Umgebung eines MRT-Gerätes abgeschirmt sind.

4. Implantierbares elektronisches Gerät gemäß Anspruch 1 bis 3, **dadurch gekennzeichnet, dass** die in der Speichereinheit (80) gespeicherten, auszuwählenden Steuerprogramme oder Steuerparameter eine asynchrone Stimulation in einem asynchronen Stimulationsmodus wie V00 oder D00 bewirken.

5. Implantierbares elektronisches Gerät gemäß Anspruch 1 bis 3, **dadurch gekennzeichnet, dass** die in der Speichereinheit (80) gespeicherten, auszuwählenden Steuerprogramme oder Steuerparameter eine Abgabe von Stimulationsimpulsen unterdrücken.

6. Implantierbares elektronisches Gerät gemäß wenigstens einem der Ansprüche 1 bis 5, **dadurch gekennzeichnet, dass** die Stimulationssteuereinheit (54) ausgebildet ist das ausgewählte Steuerprogramm oder die ausgewählten Steuerparameter solange beizubehalten, solange die Felderkennungseinheit (200) die Abwesenheit solcher elektromagnetischer Felder anzeigt, die typischerweise in der Umgebung eines Patienten außerhalb der Umgebung eines MRT-Gerätes auftreten und in der Umgebung eines MRT-Gerätes abgeschirmt sind.

7. Implantierbares elektronisches Gerät gemäß wenigstens einem der Ansprüche 1 bis 6, **dadurch gekennzeichnet, dass** das implantierbare elektronische Gerät ein implantierbarer Cardioverter/Defibrillator (ICD) und/oder ein Herzstimulator für eine kardiale Resynchronisationstherapie (CRT) ist.

8. Implantierbares elektronisches Gerät gemäß wenigstens einem der Ansprüche 1 bis 7, **dadurch gekennzeichnet, dass** das implantierbare elektronische Gerät einen Magnetfeldsensor und/oder einen Gradientenfeldsensor und/oder einen Hochfrequenz-Feldsensor und/oder einen Lagesensor und/oder einen (Lorenz-)Vibrationssensor und/oder einen Spannungsverlaufssensor aufweist, der charakteristische Spannungsverläufe beispielsweise in den Elektrodenleitungen überwacht.

## Claims

1. An implantable electronic device having at least one electromagnetic field detection unit (200), which electromagnetic field detection unit is connected to at least one antenna and is configured to respond to electromagnetic fields that typically occur in the environment surrounding a patient outside the environment of an MRI device and are shielded in the environment of an MRI device, wherein the field detection unit is also configured to indicate the absence of such electromagnetic fields, wherein the implantable electronic device is a heart stimulator (100), which has a stimulation control unit (54) and a memory unit (80), in which there is stored program information concerning control programs and/or control parameters controlling the function of the stimulation control unit (54), **characterised in that** the stimulation control unit (54) is configured to select a control program or control parameters that allows/allow safe operation of the implantable electronic device in the environment of an MRI device when the field detection unit (200) indicates the absence of electromagnetic fields that typically occur in the environment surrounding a patient outside the environment of an MRI device and are shielded in the environment of an MRI device.

2. The implantable electronic device according to claim 1, **characterised in that** the field detection unit (200) contains at least one receiver adapted to electromagnetic fields that typically occur in the environment surrounding a patient outside the environment of an MRI device and are shielded in the environment of an MRI device.

3. The implantable electronic device according to claim 1, **characterised in that** the field detection unit has a tunable receiver (230) for scanning a wider frequency range and is connected to a field characteristic memory or comprises such a memory, wherein the field characteristic memory contains characteristic features of electromagnetic fields that occur only outside the environment of an MRI device and are shielded in the environment of an MRI device.

4. The implantable electronic device according to claim 1 to 3, **characterised in that** the control programs or control parameters stored in the memory unit (80) and to be selected cause an asynchronous stimulation in an asynchronous stimulation mode, such as V00 or D00.

5. The implantable electronic device according to claim 1 to 3, **characterised in that** the control programs or control parameters stored in the memory unit (80) and to be selected suppress a delivery of stimulation pulses.

6. The implantable electronic device according to at least one of claims 1 to 5, **characterised in that** the stimulation control unit (54) is configured to maintain the selected control program or the selected control parameters as long as the field detection unit (200) indicates the absence of electromagnetic fields that typically occur in the environment surrounding a patient outside the environment of an MRI device and are shielded in the environment of an MRI device.

7. The implantable electronic device according to at least one of claims 1 to 6, **characterised in that** the implantable electronic device is an implantable cardioverter/defibrillator (ICD) and/or a heart stimulator for cardiac resynchronisation therapy (CRT).

8. The implantable electronic device according to at least one of claims 1 to 7, **characterised in that** the implantable electronic device has a magnetic field sensor and/or a gradient field sensor and/or a high-frequency field sensor and/or a position sensor and/or a (Lorenz) vibration sensor and/or a voltage profile sensor, which monitors characteristic voltage profiles, for example in the electrode leads.

## Revendications

1. Appareil électronique implantable qui présente au moins une unité de détection de champ électromagnétique (200) qui est reliée avec au moins une antenne et est conçue pour s'appliquer à des champs électromagnétiques qui se produisent de manière typique dans l'environnement d'un patient en dehors de l'environnement d'un appareil d'imagerie par résonance magnétique et sont soumis à une protection dans l'environnement d'un appareil d'IRM, où l'unité de détection de champ est en outre conçue pour indiquer l'absence de tels champs électromagnétiques, où l'appareil électronique implantable est un stimulateur cardiaque (100) qui présente une unité de commande de stimulation (54) et une unité de mémoire (80), dans laquelle sont stockés des informations de programme pour des programmes de commande et/ou des paramètre de commande qui commandent la fonction de l'unité de stimulation (54),
**caractérisé en ce que**
l'unité de stimulation (54) est conçue pour, lorsque l'unité de détection (200) indique l'absence de tels champs électromagnétiques, qui se produisent de manière typique dans l'environnement d'un patient en dehors de l'environnement d'un appareil d'IRM et sont soumis à une protection dans l'environnement d'un appareil d'IRM, choisir un programme de commande ou des paramètre de commande tels qui permettent un fonctionnement sécurisé de l'appareil électronique implantable dans l'environnement d'un appareil d'IRM.

2. Appareil électronique implantable selon la revendication 1, **caractérisé en ce que** l'unité de détection de champ (200) contient au moins un récepteur adapté à de tels champs électromagnétiques, comme ils se produisent de manière typique dans l'environnement d'un patient en dehors de l'environnement d'un appareil d'IRM, et sont soumis à une protection dans l'environnement d'un appareil d'IRM.

3. Appareil électronique implantable selon la revendication 1, **caractérisé en ce que** l'unité de détection de champ présente un récepteur (230) accordable pour le balayage d'un domaine de fréquences plus large et qui est relié avec une mémoire de caractéristiques de champs ou présente une telle mémoire, où la mémoire de caractéristiques de champs contient des propriétés caractéristiques de champs électromagnétiques qui ne se produisent qu'en dehors de l'environnement d'un appareil d'IRM et sont soumis à une protection dans l'environnement d'un appareil d'IRM.

4. Appareil électronique implantable selon les revendications 1 à 3, **caractérisé en ce que** les programmes de commande ou les paramètres de commande stockés dans l'unité de mémoire (80) sélectionnables provoquent une stimulation asynchrone dans un mode de stimulation asynchrone comme V00 ou D00.

5. Appareil électronique implantable selon les revendications 1 à 3, **caractérisé en ce que** les programmes de commande ou les paramètres de commande stockés dans l'unité de mémoire (80) sélectionnables étouffent une délivrance d'impulsions de stimulation.

6. Appareil électronique implantable selon au moins l'une des revendications 1 à 5, **caractérisé en ce que** l'unité de commande de la stimulation (54) est conçue pour conserver le programme de commande choisi ou les paramètres de commande choisis aussi longtemps que l'unité de détection de champ (200) indique l'absence de tels champs électromagnétiques, qui se produisent de manière typique dans l'environnement d'un patient en dehors de l'environnement d'un appareil d'IRM et sont soumis à une protection dans l'environnement d'un appareil d'IRM.

7. Appareil électronique implantable selon au moins l'une des revendications 1 à 6, **caractérisé en ce que** l'appareil électronique implantable est un cardioverteur / défibrillateur implantable (ICD) et/ou un stimulateur cardiaque pour une thérapie de resynchronisation cardiaque (CRT).

8. Appareil électronique implantable selon au moins l'une des revendications 1 à 7, **caractérisé en ce que** l'appareil électronique implantable présente un capteur de champ magnétique, et/ou un capteur de champ en gradient, et/ou un capteur de champ haute fréquence, et/ou un capteur de position, et/ou un capteur de vibrations de Lorentz, et/ou un capteur d'évolution de la tension, qui surveille des évolutions de tensions caractéristiques, par exemple, dans les lignes d'électrodes.
